# EUROPEAN PATENT APPLICATION

(11) **EP 1 146 337 A1**
(43) Date of publication of application: **17.10.2001**
(21) Application number: 99973328.0
(22) Date of filing: 02.12.1999
(51) Int. Cl.: G01N 33/50, G01N 33/15, C12N 15/10, A61K 45/00, A61K 39/395, A61K 31/557, A61K 48/00, A61K 31/70

(54) **PGT AND APOPTOSIS**

(30) Priority: 04.12.1998 JP 36188498; 16.12.1998 JP 37573998
(71) Applicant: WELFIDE CORPORATION, Osaka 541-0046 (JP)
(72) Inventor: KAWAMURA, Tooru c/o Welfide Corporation, Osaka-shi, Osaka 541-0046 (JP); HORIE, Satoshi, c/o Welfide Corporation, Iruma-shi, Saitama 358-0026 (JP); AKIRA, Toshiaki, c/o Welfide Corporation, Hirakata-shi, Osaka 573-1153 (JP); NAKAMURA, Norifumi, c/o Welfide Corporation, Osaka-shi, Osaka 541-0046 (JP); MARUYAMA, Tomoyuki, c/o Welfide Corporation, Hirakata-shi, Osaka573-1 153 (JP); HAYASHI, Kazutaka, c/o Welfide Corporation, Hirakata-shi, Osaka 573-1153 (JP)
(74) Representative: Forstmeyer, Dietmar, Dr. rer. nat., Dipl.-Chem.
(86) International application number: JP9906766
(87) International publication number: WO0034778

(57) **Abstract**

A method for screening an apoptosis inhibitor characterized by screening a candidate via an effect on PGT. A cell protecting agent containing as the active ingredient a substance which can be incorporated into cells via prostaglandin transporter (PGT); and a screening method therefor comprising measuring the uptake into cells via PGT. An apoptosis inducer containing as the active ingredient a substance having a PGT inhibitory effect; and a screening method therefor comprising measuring the ability to induce apoptosis of cells with the expression of PGT. Because of having a cell apoptosis inhibitory effect, the cell protecting agent is useful as a nerve cell apoptosis inhibitor, a nerve cell protecting agent, etc. and applicable to the prevention or treatment of nervous diseases, etc. The apoptosis inducer is useful in preventing and/or treating diseases in association with cell proliferation such as tumor, etc.

## Description

### Technical Field

The present invention relates to apoptosis regulators (regulating substance), cytoprotectants, apoptosis-inducing agents and methods of screening the sames, and the use the sames.

### Background Art

Prostaglandin transporter (hereinafter referred to as "PGT") possesses the property of carrier-mediated transport of prostaglandins in living body. Prostaglandins permeate biological membranes through PGT, and show (biological activity) in the cells. Or they undergo metabolic decomposition by enzyme and the like. PGT is known to be present in lungs, kidneys, brain and the like in living body; in addition, the genetic sequence that codes for PGT has also been confirmed (Science, vol. 268, pgs. 866-869, 1995).

A prostaglandin (hereinafter referred to as "PG") is one of the substances that permeate (tunneling) this PGT. PGs are known to exist in numerous types; inparticular, prostaglandin E₁ (PGE₁) and prostaglandin E₂ (PGE₂) that have high permeation velocities (Km) in permeation testing of pulmonary PGT have been reported (refer to the aforementioned document).

PGs as autacoids show a variety of biological activities in living body. Of these activities, the protective activity for neurons like amelioration of neuropathy and the suppression of neuron apoptosis have been reported (Refer to Japanese Patent Application Laid-open No. 277222/1996 gazette). Nonetheless, the mechanism of the activity relating to protective action for these PGs and cells, and especially that for neurons, is not entirely clear.

In recent years, as relates to the death of cell tissue, apoptosis (also pronounced apotosis) has garnered attention. Apoptosis means self-destruction or self-induced cell death. This apoptosis differs from necrosis, which is pathological cell death; apoptosis, is thought to be programmed as genetic information and the active death of the cell itself. Namely, it is thought that some external or internal factors become the trigger, the signal that induces apoptosis is activated, the cell itself actively decomposes, and this lead to death. The relationship between PGT and apoptosis has not been reported.

### Disclosure of the Invention

It is an object of the present invention to provide a variety of techniques using the relationship between PGT and apoptosis; namely, technology relating to suppress or induction of apoptosis through PGT, screening of substances that regulate apoptosis using PGT, use of screened substances, and culture of PGT-expressing cells using substances that suppress apoptosis. The present inventors are the first to confirm the relationship between PGT and apoptosis as a result of advanced research in light of the aforementioned circumstances. Namely, the inventors are the first to succeed in elucidating the mechanism leading to apoptosis through PGT. Specifically, the present invention has been led to completion that can protect cells and suppress apoptosis by adapting substances that positively affect PGT and can conversely induce apoptosis by adapting substances that negatively affect PGT.

Namely, the method of screening apoptosis regulators in the present invention is characterized by screening candidates through their activity on PGT. Herein, the activity on PGT may be measured by the amount of uptake into cells through PGT. In addition, the activity on PGT may be measured by way of the velocity of uptake into cells through PGT.

The method of screening apoptosis regulators in the present invention may be a method with measurement by activity on PGT with an inhibitory effect on the activity of PGT or the expression of PGT.

The method of screening apoptosis regulators in the present invention may be a method characterized by screening of substances having activity on PGT and substantially no hypotensive effect.

The apoptosis regulators in the present invention are substances screened by the method of screening regulators in the present invention.

The cytoprotectant in the present invention is composed of, as an active ingredient, an apoptosis regulator having an activity with itself be taken into cells through PGT and an activity to suppress apoptosis. Herein, as for the activity of uptake into cells through PGT, the cytoprotectant may display an amount of uptake of at least about 70 fmol/mg protein/10 mins. In addition, as for the activity of uptake into cells through PGT, the cytoprotectant may be one having affinity for PGT displayed by a permeation velocity (Km) of no more than about 100 nM. Moreover, the cytoprotectant in the present invention may have activity with respect to encephalon cells, neurons, or kidney cells. Furthermore, a cytoprotectant may be characterized by having substantially no hypotensive effect. The apoptosis-inducing agent in the present invention is composed of, as an active ingredient, an apoptosis regulator having an inhibitory effect on PGT expression or PGT activity and an activity to induce apoptosis. Herein, the apoptosis regulator may be anti-PGT antibodies or PGT antisense.

The cytoprotectant in the present invention is an one which is a apoptosis regulator selected from PGK₁, PGK₂, or bicyclo PGE₂.

The method of culturing cells expressing PGT in the present invention is characterized by using a culture medium with addition of a cytoprotectant being composed of, as an active ingredient, an apoptosis regulator having activity to suppress apoptosis. Herein, the added cytoprotectant is characterized by having activity with itself be taken into cells through PGT. In addition, the characteristic point is that the added cytoprotectant herein is PGE₁.

The method of regulating apoptosis in the present invention is characterized by administration of an effective dose of an apoptosis-inducing agent or a cytoprotectant according to the present invention.

The use of an apoptosis-inducing agent in the present invention is characterized by the use of an apoptosis-inducing agent or a cytoprotectant prepared according to the present invention with the aim of manufacturing medicament for regulating apoptosis.

### The preferred mode for carrying out the invention

The present invention will hereafter be described in detail.

### (A) Amethod for screening of apoptosis regulators (apoptosis-regulating substances)

The method for screening apoptosis regulators (apoptosis-regulating substances) in the present invention is characterized by a screening of desired substances using activity on PGT as an indicator of candidate. Apoptosis regulators mean substances having apoptosis-suppressing activity or substances having apoptosis-inducing activity. The apoptosis regulators in the present invention are substances having activity to suppress or induce PGT activity or its expression.

### 1) Method of screening substances having apoptosis suppressing activity

The method of screening substances having apoptosis-suppressing activity in the present invention comprises screening substances having the property of uptake into cells through PGT, preferably screening substances having an affinity to PGT and having the property of uptake into cells through PGT. Herein, "having an affinity for PGT" specifically means that Km is no more than about 100 nM in systems for measurement of cell uptake using cells with forced expression of hPGT, for example, HeLa cells. In addition, "uptake into cells through PGT" specifically means to be, for example, a level of no less than 70 fmol/mg protein/10 mins in systems for measurement of the radioactivity in cells after 10 mins of incubation at 37°C with tritium labeling using differentiated PC12 cells. Substances satisfying these conditions will be screened. Exemplary specific methods include the aforementioned methods of screening with either permeation velocity in PGT-expressing cells or an amount of uptake of an apoptosis-suppressing effect and the like as indication, but the method is not limited by those mentioned herein. Furthermore, a screening of substances having substantially no hypotensive effect is more preferable.

### 2) Method of screening substances having apoptosis-inducing activity

The method of screening substances having the apoptosis-inducing activity in the present invention comprises screening substances having inhibitory activity on PGT and substances having activity to suppress the expression of PGT itself. Specific example includes, for example, using PGT-expressing cells such as HeLa cells that forcibly express hPGT, and then screening substances that are able to induce cell apoptosis. In addition, substances where uptake into cells through PGT is known, for example PGE₁ and the like, labelling with radioactive isotopes; screening of substances that reduce or eliminate the permeation velocity or amount of uptake into PGT-expressing cells and then obtained substances having apoptosis-inducing action. The method of screening substances having apoptosis-inducing activity in the present invention may be a method to allow a screening of substances having activity to repress the expression of PGT itself as well as substances having PGT-inhibiting activity, but it is not limited by the aforementioned examples. Furthermore, a screening of substances having substantially no hypotensive effect is even more preferable.

### (B) Cytoprotectant

The cytoprotectant in the present invention is composed of, as an active ingredient, a substance having the property of uptake into cells though PGT and preferably an active ingredient having an affinity for PGT and having a property of uptake into cells through PGT. Herein, "having an affinity for PGT" specifically may be Km of no more than about 100 nM in systems for measurement of uptake into cells using HeLa cells that forcedly express hPGT. In addition, "uptake into cells through PGT" specifically may be a level of no less than about 70 fmol/mg protein/10 mins in a system for measurement of the radioactivity in cells after 10 mins of incubation at 37°C with tritium labeling using differentiated PC12 cells.

The cytoprotectant in the present invention may include substances screened by the aforementioned (A) 1) a method of screening substances having apoptosis-repressing activity. Said substances have apoptosis-repressing activity. These substances may be manufactured by chemical synthesis, extraction and isolation, or genetic engineering and may be provided as pharmaceuticals by usual preparation techniques.

Examples of these substances include PGE₁, PGE₂, PGF_{2α}, PGD₂, PGK₁, PGK₂, bicyclo PGE₂, or their active derivatives, their precursors, for example, alkyl esters (Japanese Patent Application Laid-open No. 216820/1984), alkoxy-carbonyl alkyls, alkyl-carbonyl oxy-alkyl esters (Japanese Patent Application Laid-open No. 206344/1984,13779/1985), 7 ― thios (Japanese Patent Application Laid-open No. 110562/1983), 9-acyloxys (Japanese Patent Application Laid-open No. 39660/1983, 204853/1991, 213862/1993), or substances having the same level of PGT affinity and having a property of uptake into cells through PGT and the like, and preferably new substances having the property of uptake into cells through PGT or known substances wherein having a property of uptake into cells through PGT was not known.

In particular, PGK₁, PGK₂, and bicyclo PGE₂ have displayed activity to suppress apoptosis of neurons in the rat cerebral cortex by amyloid beta peptide. The present activity is an apoptosis- suppressing action through PGT. PGK₁, PGK₂, and bicyclo PGE₂ are known substances, but their apoptosis-suppressing activity through PGT has yet to be reported. Furthermore, PGE₁ is known to have hypotensive effect and its adverse effects when used as pharmaceutical composition are a problem. However, PGK₁, PGK₂, and bicyclo PGE₂ have been determined to have potential in sufficiently suppressing apoptosis in a dose that does not substantially cause a decrease in blood pressure, and this usefulness is superior to PGE₁.

Said substances have cytoprotective activity, and protective activity for neurons in particular, specifically having activity with respect to neuro degeneration and activity to suppress apoptosis induction in neurons, being useful as a cytoprotectant for neurons. Examples of related disorders include neurological disorders, disorders accompanied by neurodegeneration, Alzheimer's disease, Parkinson's disease, Huntington's chorea, amyotrophic lateral sclerosis, and spinal canal stenosis etc. These substances may be useful in the prevention, treatment, and remediation of these disorders.

In addition, said substances may be useful as a cytoprotectant for kidney cells. These substances may be useful in the prevention, treatment, and remediation of renal disorders such as nephritis, renal failure, glomerulonephritis, and nephrotic syndrome. In particular, because of repression of cell injury by NO, said substance may be useful for the prevention and treatment of renal disorders associated with radicals and apoptosis such as acute renal failure, drug-inducedrenal failure, and chronic renal failure.

Moreover, said substances, PGE₁ and the like, because of having cytoprotective activity, may effectively act for survival and prolong of PGT-expressing cells by its addition to a cell culture system. In particular, long-term culturing in may be possible for distressed cells of brain cells, neurons and the like.

As exemplary formulations of said substances, administration in living organisms by way of preparations of ethanol solutions, liposomes, lipid emulsions, cyclodextrin - inclusion compound and the like may be possible. Moreover, usual formulation techniques may be used as necessary.

Preparation of an ethanol solution may be performed by way of dissolving said substances in ethanol. Furthermore, said ethanol solution may be used by dilution with physiological saline or a glucose solution prior to medical use.

Preparation of liposomes, for example, can be performed by dissolving phospholipids in an organic solvent (chloroform and the like), adding to said solution, a solution where said substances are dissolved in a solvent (ethanol and the like), evaporating the solvents, adding phosphate buffer to this, shaking, ultrasonic treating and centrifugating, and then collection the supernatant by means of filter treatment.

Preparation of a lipid emulsion, for example, can be performed by mixing said substance, an oil constituent (vegetable oils like soybeans oil , sesame seed oil, and olive oil, MCT and the like), emulsifying agent (phospholipids and the like) and the like, heating the mixture to form a solution, adding the required amount of water to it, and then emulsifying and homogenizing it by using an emulsifying machine (a homogenizer, for example, one of a high-pressure jet type-, an ultrasonic type- and the like). In addition, freeze-drying of this resultant may be possible. Furthermore, an emulsifying adjuvant may be added for lipid emulsion. Examples of the emulsifying adjuvant include, for example, glycerin and saccharides (for example, glucose, sorbitol, fructose and the like).

Preparation of Cyclodextrin - inclusion compound, for example, can be performed by dissolving said substance in a solvent (ethanol and the like), adding to said solution, a solution where cyclodextrin is heat-dissolved in water and the like, cooling it, filtering deposited precipitation and then dry-sterilizing. To this end, the cyclodextrin used is in proportion to the size of said substances, and cyclodextrin with different void diameters (α, β, and γ) may be selected arbitrarily.

The dose of said substances may be selected arbitrarily in accordance with the patient's condition, sex, age, and body weight. As for the route of administration, oral and parenteral administration may be performed. Preferably, examples thereof include intravenous administration as the form of injection. Examples of the dose include a dose of about 1-1000 µg per day.

### (C) Apoptosis-inducing agents

The apoptosis-inducing agent in the present invention is composed of, as an active ingredient, substances having activity to inhibit PGT, that is, substances having activity to suppress PGT expression or PGT activity. Exemplary substances include PGT antisense, anti-PGT antibodies and the like.

In addition, the apoptosis-inducing agent in the present invention may be a substance screened by the aforementioned (A) (2) method of screening substances having apoptosis-inducing activity. Said substances have activity to suppress PGT expression or PGT activity. These substances may be manufactured separately by synthesis, extraction and isolation, or genetic engineering and may be provided as pharmaceuticals by way of usual formulation techniques.

### (1) PGT antisense

PGT antisense is a genetic sequence having the complementary relationship with genes that code for PGT, and additionally as long as the antisense can subsequently inhibit PGT by changing places with genes that code for PGT on DNA strands in the cell nucleus, it is acceptable. Specifically, examples include oligonucleotides having DNA sequences of 5'-GGCTTGAGCAGGAGCCCCAT-3' and the like. These can be prepared by usual DNA synthesis methods. In addition, these substances may be thioate-modified.

As for the PGT antisense, preferably, administration in living organisms may be possible by preparations of mixtures with Lipofectin and the like, liposomes, lipid emulsions, cyclodextrin-inclusion compound. In addition, these composition may be used after binding with angiogenesis factor (for example, angiopoietein, aminopeptidase A and the like) for heightened specific affinity and/or clustering for cancer cells. Moreover, usual formation techniques may be used as necessary.

### (2) PGT antibodies

No particular limitation is imposed on the PGT antibodies so long as they can be recognized as an antigen for PGT. The PGT antibodies may be polyclonal antibodies or monoclonal antibodies, and related PGT antibodies may be prepared in accordance with usual methods. For example, polyclonal antibodies may be prepared by way of collecting and purification of antiserum of animals immunized with PGT. In addition, monoclonal antibodies can be produced by way of culturing hybridoma prepared with cell fusion of proliferative cells like myeloma cells and spleen cells of animals immunized with PGT or by culturing transformants obtained by transforming said spleen cells with EB virus (Journal of Chemical Investigation, vol.98, no.5, pgs. 1142-1149, 1996; US Patent No. 5792851 and the like).

In addition, examples of the PGT antibodies include chimera antibodies or humanized antibodies. Chimera antibodies can be prepared by matching constant regions of human antibodies with variable regions of the aforementioned non-human monoclonal antibodies. Humanized antibodies among the aforementioned chimera antibodies are antibodies where amino acids of variable regions for humans permit substitution (not causing new antigenicity). These may be prepared by genetic engineering

Formations containing the PGT antibodies may be prepared in accordance with common formulation methods.

The dose of substances having PGT-inhibiting activity in the present invention can be selected arbitrarily in accordance with the patient's condition, sex, age, and body weight. For example, examples of the dose for an adult patient include a dose of about 0.001-1000 mg/day. As for the route of administration, oral and parenteral routes may be used.

The substances having PGT-inhibiting activity in the present invention are those having apoptosis-inducing activity and may be useful in prevention and/or treatment of disorders that occur by way of tumor or pathogenic cells. For example, these substances may be useful as antitumor agents, anticancer agents, and antitumor drugs or in the prevention and/or treatment of proliferative disorders other than tumors, and moreover in disorders where various types of viral infections are the cause, infectious diseases resulting from the HIV virus, and acquired immune deficiency syndrome (AIDS) in particular.

### [Embodiments]

Example and experiments will be mentioned to more specifically describe the present invention but the present invention is in no way limited by those mentioned herein.

### [Example 1] Cyclodextrin - inclusion compound

A solution prepared by heat-dissolution in 6 ml water of 257 mg β cyclodextrin was added to a solution where 17 mg PGE₁ is dissolved in 0.2 ml ethanol. After mixing at 45°C, it was returned to room temperature and a precipitate was deposited. This was kept at 0°C overnight and then filtered. After washing with a 50% ethanol aqueous solution, Cyclodextrin - inclusion compounds were obtained by way of dry - sterilization.

### [Example 2] Preparation of liposomes

After dissolving 60 mg egg yolk phosphatidylcholine and 11 mg oleyl amine in 5 ml chloroform, a solution of 30 mg PGE₁ in 100 µl ethanol was added. Placed in an Erlenmeyer flask, the solution was evaporated with a rotary evaporator. To this was added 1 ml of 0.1 M phosphate buffered saline (pH 5); after shaking, ultrasonic treatment (sonicate), and centrifugation, the liposome preparation was obtained by filtering the supernatant with a 0.2 µm membrane filter.

### [Example 3] Ethanol solution

An ethanol solution was obtained by dissolving 500 µg PGE₁ in 1 ml ethanol. Before medical use, this solution was diluted using physiological saline or glucose solution.

### [Example 4] Lipid emulsion

To 30 g purified soybean oil was added 5.4 g highly purifined egg yolk phospholipids, 1.5 mg PGE₁, and 0.72 g oleic acid for heat dissolution at 40-75°C. To this was added 200 ml distilled water. Next, 7. 5 g glycerin (Pharmacopoeia Japonica) was added. The total amount was brought to 300 ml with distilled water at 20-40°C, and rough emulsification was conducted with a homogenizer. High-pressure emulsification was performed with a Manton-Gaulin model homogenizer and lipid emulsion with homogenized and having fine particle was obtained. The average particle diameter of this emulsion was 0.15-0.4 µm, and particles larger than 1 µm were not contained therein.

### [Example 5] Lipid emulsion

PGE₂ was used in place of PGE₁, but other manners were in accordance with Example 4, and a lipid emulsion was prepared.

### [Example 6]

As an antisense-oligonucleotide specificto PGT, the DNA sequence yielded by 5'-GGCTTGAGCAGGAGCCCCAT-3' was synthesized using a DNA synthesizer. Next, it was modified by Thioate. Then, it was mixing with equivalent amount of Lipofectin (Gibco).

### [Example 7]

PGT or fragments thereof with an equivalent amount of adjuvant were administered to rabbits multiple times in a 2-3-day period. Afterwards, the antiserum was collected. The PGT antibodies were prepared by purifying the antiserum in accordance with usual methods.

### [Example 8]

PGK₁, PGK₂, or bicyclo PGE₂ was used in place of PGE₁, but other manners were in accordance with Example 3, and an ethanol solution was prepared.

### [Example 9]

Anti-PGT antibodies were prepared by using a conjugate of rat PGT N terminal fragments (Corresponding to N terminals 1-23. Composing of an amino acid sequence (MGLLLKPGAR QGSGTSSVPD RRC) and keyhole limpet-hemocyanin (KLH) as an immunogen.

Immunization was performed by an immunogen with equivalent amount of Freund's complete adjuvant (or incomplete adjuvant) that was injected in a single administration of 1 mg to domestic rabbits. In the following 2-week interval, a total of 3 additional immunizations were performed. An antiserum was obtained with exsanguination after 2 months.

The antiserum was diluted to 2x with 0.02 M isotonic phosphate buffered saline (pH 7, hereafter PBS), and a saturated ammonium sulfate solution was added to yield a 40% concentration of saturated solution. Centrifugation was performed after standing, and then the precipitating fraction was collected and dissolved in PBS. A saturated ammonium sulfate solution was added to yield a 40% concentration of saturated solution. Centrifugation was performed after standing, and then the precipitating fraction was collected and dissolved in PBS. Dialysis was performed with water in a dialysis tube, and the ammonium sulfate was removed. And then purification was performed by affinity chromatography using a carrier where the aforementioned PGT fragment was fixed on agarose. That is antibody fractions dissolved in PBS were lodged on a column. Washing was performed with PBS containing 1 M sodium chloride, and elution and collection of the antibody fraction was performed with 4 M magnesium chloride solution. Pure antibodies were produced after dialysis with PBS. Measurement of the antibody concentration was conducted using a Protein Assay Kit from Bio-Rad Laboratories and the concentration of the pure antibodies obtained was about 8 µg/ml.

### [Experiment 1]

### 1) Suppression of apoptosis in neurons

(1) After growing rat adrenal pheochromocytoma PC12 cells (obtained by: ATCC; importer: Dainippon Pharmaceutical) with RPMI1640 containing 10 ml/l heat-inactivated horse serum and 5 ml/l heat-inactivated fetal calf serum, culturing was performed with RPMI1640 containing mouse β NGF (100 ng/ml), N2 supplement, and TIP (5 µg/ml transferrin, 5 µg/ml insulin, and 10 ng/ml progesterone). Differentiation to neuron - like cells was performed.
(2) Differentiated PC12 cells were washed with Neurobasal culture medium. After replacing the culture medium with RPMI1640 not containing βNGF, N2 supplement, and TIP, mouse β NGF antibodies (final concentration: 50 ng/ml) were added. By culturing for 24hrs, apoptosis was induced.
(3) A PGE₁ ethanol solution was added (final concentration: 0.01-1 µM) to fresh culture medium during medium replacement and culturing was performed for the same amount of time. As a negative control, a solvent (ethanol) alone was added in the same manner and culturing was performed for the same period of time. Apoptosis detection was performed by Hoechst 33342 staining (at 1 mM with a reaction in 2 minutes at room temperature) after fixation (room temperature for 30 mins) of cells with a 1% glutaraldehyde solution. Observation was made from 5 arbitrary visual fields using a fluorescence microscope. Total cell and apoptotic cells were counted, and incident of apoptosis was calculated.
(4) The values obtained were expressed as means±SEM. As for statistical analysis, a student's t-test (hereafter noted as a t-test) was used for two groups and Dunnet's test was used after one-way layout dimensional analysis (one-way ANOVA) for numerous groups; critical values for both were considered to be a significant difference below 5%. Results are indicated in Table 1.

As indicated in Table 1, it was found that PGE₁, have suppressed cell apoptosis at 1 µM.

**[Table 1]**

| PGE₁ concentration | Incidence of apoptosis (%) | Statistical analysis |
|---|---|---|
| Prior to the experiment | 4.6±1.0 | |
| None (negative control) | 26.3±1.8 | |
| 0.01 µM | 21.3±1.5 | |
| 0.1 µM | 19.1±8.1 | |
| 1 µM | 17.1±0.6 | * |

| | | |
|---|---|---|
| *Indicates a significant difference in comparison to the negative control. | | |

### 2) Apoptosis and PGT expression

Total RNA from PC12 cells was extracted and isolated using TRizo (Gibco). The primers with correspond to PGT used with reverse transcription - polymerase chain reaction (RT-PCR) were designed with reference to a rat PGT cDNA sequence (Kanai, N. et al., Science, vol.268, 866-869, 1995). The PGT primers used are indicated as follows:

Sense (starting base number: 264)
5'-GAGCAGTCTCACCACAATCG-3'

Antisense (starting base number: 670)
5'-GGCTCGGCAAAGTCATCCAC-3'

The molecular weight of a PCR product as amplified by using primer pairs was calculated to be 444 bp. RT-PCR was performed using a PCR kit (Takara Co. Ltd.). DNA replication was performed in 25 cycles with a program of 1 min at 95°C, 2 min at 60°C, and 3 min at 72°C using a DNA thermal cycler (PJ 2000, PerkinElmer Inc.). Using 1/5 of the amount of the sample2% agarose electrophoresis was performed for individual RT-PCR products after reacting, and after ethidium bromide staining analysis was performed using ultraviolet irradiation.

With PC12 cells, 1 strand of genetic fragments derived from mRNA were amplified. Thus, transcription specific to PGT was confirmed.

### [Experiment 2]

As regards the apoptosis- suppressing activity of PGE₁ in nerve neurons, the manners other than TUNEL staining for detection of apoptosis were in accordance with ones in Experiment 1. The results are indicated in Table2. As indicated in Table2, PGE₁ 0.1 µM and 1 µM was confirmed to have suppressed cell apoptosis at 0.1 µM and 1 µM.

**[Table 2]**

| PGE₁ concentration | TUNEL-positive cells (%) | Statistical analysis |
|---|---|---|
| Prior to the experiment | 7.00±1.27 | |
| None (negative control) | 30.93±2.31 | |
| 0.1 µM | 13.80±0.75 | **P=0.0006 |
| 1 µM | 8.10±1.57 | **P=0.0001 |

| | | |
|---|---|---|
| **Indicates a significant difference in comparison to the negative control. | | |

### [Experiment 3]

1) Differentiated PC12 cells and tritium-labelled PGE₁ (added concentration: 0.1 µM) were subjected to 10 mins of incubation at 37°C. Afterwards, the cells were washed with ice-cold phosphate buffered saline containing 5 g/l of bovine serum albumin and next washed with ice-cold phosphate buffered saline. Then, radioactivity was measured. The amount of PGE₁ uptake into PC12 cells was calculated.
2) Bromcresol green (hereinafter noted as "BrCG"), which is a PGT inhibitor, was placed together in the culture medium at the addition of PGE₁. Afterwards, treatment was conducted in the same manner as mentioned in 1). The results are indicated in Table 3.

**[Table 3]**

| Amount of BrCG addition | Amount of PGE₁ uptake (fmol/mg protein/10 mins) |
|---|---|
| No addition | 78±11 |
| 1 µM | 60±15 |
| 1 µM | 20±8* |
| Values are expressed as means±SEM. | |

| | |
|---|---|
| *Indicates a critical value of less than 5% in the t-test as a significant difference in relation to the PGE₁ addition group. | |

3) The antisense oligonucleotide specific to PGT cDNA was designed as follows: 5′-GGCTTGAGCAGGAGCCCCAT-3′
Moreover, said antisense was thioate modified. Using Lipofectin (Gibco), PC12 underwent transfection during 2 days prior to the addition of PGE₁. Afterwards, treatment was conducted in the same manner as mentioned in 1). The results are indicated in Table 4.

**[Table 4]**

| Amount of antisense added | Amount of PGE₁ uptake (fmol/mg protein/10 mins) |
|---|---|
| No addition | 78±11 |
| 0.5 µM | 56±17 |
| 1.5 µM | 14±14* |
| Values are expressed as means±SEM. | |

| | |
|---|---|
| *Indicates a critical value of less than 5% in the t-test as a significant difference in relation to the PGE₁ addition group. | |

4) Intercellular signals via SAPK/JNK
Stress-activated protein kinase/Jun N-terminal kinase (abbreviated SAPK/JNK) is associated with apoptosis induction. Thus, intercellular signals within cells via SAPK/JNK were investigated based upon effects with respect to variations in SAPK/JNK activity.
The present experiment was performed with Western blot analysis. Individual additives were added to cell culturing medium for PC12 cells in accordance with the aforementioned experiment. Culturing was performed for 1-4 hrs after withdrawal of NGF. PC12 cells were lysed with a buffer solution, centrifuged, and the supernatant of cell lysate was prepared. After said supernatant was incubated with JNK 1 polyclonal antibodies (Santa Cruz Biotechnology) at 4°C for 1 hr. Protein A sepharose was added to it. The resultant was incubated for 1 hr and the immunoprecipitate as described below was provided. 100 mg of cell lysate was conducted with SDS-PAGE electrophoresis, and transferred to a nylon membrane (Amersham Pharmacia Biotech). Blotting of phosphorylated (active) JNK antibodies (Santa Cruz Biotechnology) was performed with this membrane, and bands were detected in accordance with the instruction manual. Intercellular signals within cells were amplified by chemiluminescence and auto radioactivity methods (ECL, Amersham Pharmacia Biotech). The results are indicated in Table 5.

**[Table 5]**

| Additive | Degree of luminescence |
|---|---|
| No addition | +++ |
| PGE₁ (1 µM) | ± |
| PGE₁ (0.1 µM) | + |
| PGE₁ (0.1 µM) + BrCG (10 µM) | +++ |
| PGE₁ (0.1 uM) + PGT antisense | +++ |
| Degree of luminescence: +++ is extremely strong. + is confirmed luminescence. ±is poor luminescence. | |

SAPK/JNK was activated at 1 hr after NGF withdrawal. In PC12 cells treated with PGE₁, an increase in the aforementioned activity was not observed and the activation was suppressed. BrCG, which is an inhibitor against PGT, or antisense inhibited PGE₁- suppressing effect with respect to SAPK/JNK activation.

From the aforementioned results, the relationship between cell apoptosis and substances having a property of uptake into cells through PGT was examined.

When said substances near PGT, are taken into cell, cell apoptosis is suppressed.

When said substances, with co-existing of inhibitors, are inhibited uptake into cells, apoptosis is not suppressed.

Based on these results, it is thought that substances having the property of uptake into cells through PGT induce various types of intercellular signals, with uptake into cells through PGT and signals that are induced directly/indirectly suppress apoptosis of cells and particularly neuron.

### [Experiment 4]

1) Rat adrenal pheochromocytoma PC12 cells (obtained by: ATCC; importer: Dainippon Pharmaceutical) was cultured in a Neurobasal culture medium containing mouse β NGF (100 ng/ml), N2 supplement (1%), and TIP (5 µg/ml transferrin, 5 µg/ml insulin, and 10 ng/ml progesterone). Differentiation of neuron was performed.
2) Then, antisense solution prepared in Example 6 is added (final concentration: 600 nM) and cultured for 2 days. As a negative control, Lipofectin in alone is added in the same manner and cultured for the same period of time. Apoptosis detection is performed by TUNEL staining after cell fixation (room temperature for 30 min) with a 10% neutral formalin buffer solution. Observation was made from 6 arbitrary visual fields using a light microscope. Total cells and TUNEL (apoptosis) - positive cells were counted, and the incidence of apoptosis was calculated. The results are indicated in Table 6.

As indicated in Table 6, it was found that apoptosis have been induced in a system with antisense addition.

**[Table 6]**

| Antisense | TUNEL-positive cells (%) |
|---|---|
| No addition | 7.4±0.9 |
| Addition | 12.9±1.1 |

### [Experiment 5]

Induction of apoptosis by amyloid beta peptide
1) Culturing of rat cerebral cortical neurons
The cortex region of embryonic day 17 or 18 rat cerebrum was extracted on ice. Cells were dispersed using a neuron dispersal solution (Sumilon) after sectioning. Afterwards, cells were dispersed to a density of 1.5x10⁵ cells/cm² in a culturing flask that was coated with polyethyleneimine beforehand. After 4 days of culturing, the following experiment was provided. Therefore, as the culture medium, Neurobasal medium (Gibco) adding with B27 supplement (1/50 volume), 2- mercaptoethanol (27.5 µM), L-glutamic acid (25 µM), and glutamine (0.5 mM) was used.
2) Induction of apoptosis by amyloid beta peptide
Amyloid beta peptide25-35 (Aβ₂₅₋₃₅) was dissolved in distilled water to a concentration of 1 mM, incubated for 1 week at about 37°C, and Aged-Aβ₂₅₋₃₅ was prepared. The induction of apoptosis in neuron was performed by the replacement of the aforementioned culturemedium (withtheexceptionofL-glutamicacid) containing 10 µM Aged -Aβ₂₅₋₃₅.
3) It was investigated whether PGT expressed in neurons at 24 hrs after induction of apoptosis. PGT in a total cell lysate was detected using antibodies against the N terminals of PGT by Western blotting. As a result, single band with a molecular weight of about 40 kd were detected. Namely, brain type PGT was determined to have a different molecular weight than previously known lung type PGT (molecular weight: 70 kd).
4) It was investigated that the suppressing effect of PGE₁ on apoptosis induced by amyloid beta peptide. After dissolution of PGE₁ in ethanol, when the aforementioned 2) Aged -Aβ₂₅₋₃₅ was added to the culture medium, simultaneously PGE₁ solution was added to it. The final concentration thereof was 1 µM.
5) Detection of apoptosis
Cells were washed with PBS (-) at 24 hrs after induction of apoptosis and fixed for 30 mins at room temperature using a 1% glutaraldehyde solution (in PBS). Next, cell were washed with PBS (-) two times and followed by incubation with 1 mM Hoechst 33342 solution (in PBS) for 2 mins. Afterwards, observation of nuclear chromatin morphology was conducted using a fluorescence microscope with 4 arbitrary visual fields. Normal cells and apoptotic cells (cell where chromatin fragmentation or condensation was appeared) were counted, and the ratio was calculated as the incidence of apoptosis. The n was 3. The results are indicated in Table 7.
6) The relationship between PGT inhibitors and apoptosis induced by amyloid beta peptide was investigated. BrCG, which is a PGT inhibitor, with a final concentration of 60 µM was added simultaneously with PGE₁ but other manners were in accordance with the aforementioned ones in 1)-5). The results are indicated in Table 7.

**[Table 7]**

| Agent | Incidence of apoptosis (%) |
|---|---|
| Vehicle | 30±2 |
| PGE₁ | 23±2** |
| PGE₁ + BrCG | 28±11^{#} |
| Values are expressed as means±SEM. | |

| | |
|---|---|
| **Indicates a critical value of less than 1% with respect to the vehicle (amyloid beta peptide treatment only) during detection in Dunnet's test. | |
| # Indicates a critical value of less than 5% in the t-test as a significant difference in relation to the PGE₁ addition group. | |

PGE₁ suppressed apoptosis inducted by amyloid beta peptide. Moreover, BrCG, which is a PGT inhibitor, inhibited said effect of PGE₁ (inhibitory effect against apoptosis induced by amyloid beta peptide). As a result, said effect of PGE₁ is suggested to generate through PGT.

### [Experiment 6]

It was investigated that the influence of PGT antisense on the inhibitory effect of PGE₁ against apoptosis induced by amyloid beta peptide. PGT antisense was used as it is with regard to the oligonucleotide in Experiment 3. The addition concentration thereof was 1.5 µM. Incidence of apoptosis was performed in accordance with Experiment 5 . The n was 3 . The results are indicated in Table 8.

**[Table 8]**

| Agent | Incidence of apoptosis (%) |
|---|---|
| Vehicle | 33±2 |
| PGE₁ | 23±2** |
| PGE₁ + PGT antisense | 32±2^{#} |
| Values are expressed as means±SEM. | |

| | |
|---|---|
| **Indicates a critical value of less than 1% with respect to the vehicle (amyloid beta peptide treatment only) during detection in Dunnet's test. | |
| # Indicates a critical value of less than 5% in the t-test as a significant difference in relation to the PGE₁ addition group. | |

### [Experiment 7]

1) Drugs having an inhibitory activity against induction of apoptosis by amyloid beta peptide were investigated. After dissolving test drugs (PGE₁, PGK₁, PGK₂, and bicyclo PGE₂) in ethanol, when Aged- Aβ₂₅₋₃₅ was added to the culture medium, simultaneously test drug solution were added to it. But other manners were in accordance with Experiment 5. Ratio of normal cells and apoptotic cells allowed calculation of the rate of inhibition of apoptosis induction. The results are indicated in Table 9.
2) The hypotensive effect of the test drugs was examined. After anesthetizing normal rats (male, body weight of about 300 g) with intraperitoneal administration of Nembutal, the dorsal region was fixed. Next, a cannula was inserted in the carotid artery and blood pressure was measured through a transducer (Nihon Kohden Corporation); simultaneous recording was made in combination with a polygraph (Nihon Kohden Corporation). After dissolving the test drugs in ethanol, and diluted (x200) with saline. Administration was given in the caudal artery in a dose of 100 µg/kg body weight. The mean blood pressure (MBP) was measured. The decrease in blood pressure was calculated from the measured values before and after administration. The n was 3 per group. The results are indicated in Table 9.

**[Table 9]**

| Test drug | Inhibition of apoptosis induction (%) | Decrease in blood pressure |
|---|---|---|
| PGK₁ | 45.4 | -5 |
| PGK₂ | 36.2 | +2 |
| Bicyclo PGE₂ | 40.6 | 0 |
| PGE₁ | 38.2 | -55 |

PGK₁, PGK₂, and bicyclo PGE₂ were determined to potentially suppress apoptosis sufficiently in a dose that substantially causes no decrease in blood pressure.

### [Experiment 8]

1) Apoptosis was induced by NO (nitrogen monoxide) in kidney cells. NRK52E cells, which are established from a rat renal tubule epithelial cell line, mainly derived from distal were cultured for 24 hours with a chamber slide that was coated with type-I collagen. Afterwards, the cultured medium was changed to a serum-free medium, and apoptosis was induced by addition of NOC12 (0.4mM) or S-nitroso L-glutathione (GSNO: 1 mM), which are NO donors.
2) It was investigated whether PGT expressed in kidney cells where apoptosis was induced. PGT in a total kidney cell lysate solution was detected using antibodies against the N terminals of PGT by Western blotting. As a result, single band with a molecular weight of about 40 kd were detected as in brain type PGT in the kidney as well.
3) It was investigated whether PGT localized in kidney cells where apoptosis was induced. PGT in kidney tissue section was detected by histoimmunological staining using antibody against the N terminal of PGT. Strong staining was observed in distal renal tubules and collecting tubules.
4) Inhibitory effect of PGE₁ to the induction of apoptosis in kidney cells was investigated, PGE₁ was added simultaneously with the addition of a NO donor to a final concentration of 1 or 10 µM. After 24 hrs, cells were fixed with a 1% glutaraldehyde solution and stained with Hoechst 33342. Three visual fields were selected arbitrarily per slide, and the ratio of the cells count where nuclear fragmentation and condensation appeared was calculated with respect to the total cells in the field (magnification: 400x). The n was 5. The results are indicated in Tables 10 and 11.

**[Table 10]**

| (Cases that NOC12 was added) | |
|---|---|
| Amount of PGE₁ added | Incidence of apoptosis (%) |
| No addition (vehicle) | 16±1 |
| 1 µM | 11±2* |
| 10 µM | 6±1** |
| Values are expressed as means±SEM. | |

| | |
|---|---|
| *Indicates a critical value of less than 1% as a significant difference with respect to the vehicle during detection in Dunnet's test. | |
| ** Indicates the same critical value of less than 1% as a significant difference. | |

**[Table 11]**

| (Cases that GSNO was added) | |
|---|---|
| Amount of PGE₁ added | Incidence of apoptosis (%) |
| No addition (vehicle) | 25±4 |
| 1 µM | 10±2* |
| 10 µM | 11±3** |
| Values are expressed as means±SEM. | |

| | |
|---|---|
| *Indicates a critical value of less than 1% as a significant difference with respect to the vehicle during detection in Dunnet's test. | |
| ** Indicates the same critical value of less than 1% as a significant difference. | |

PGE₁ suppressed induction of apoptosis in kidney cells. Specifically, it was determined that it suppressed apoptosis inducted by GSNO in a rat renal tubule epithelial cell line (NRK52E cells).
5) The relationship between PGT inhibitors and kidney cell apoptosis induced by GSNO was examined. With a final concentration of BrCG, which is a PGT inhibitor, of 60 µM, manners other than simultaneous addition with GSNO and PGE₁ were in accordance with the aforementioned ones in 4). The PGE₁ final concentration was 1 µM. The n was 3. The results are indicated in Table 12.

**[Table 12]**

| Agent | Incidence of apoptosis (%) |
|---|---|
| No addition (vehicle; GSNO treatment only) | 19±1 |
| PGE₁ | 8±2 |
| PGE₁ + BrCG | 13±1* |
| Values are expressed as means±SEM. | |

| | |
|---|---|
| * Indicates a critical value of less than 5% in the t-test as a significant difference in relation to the PGE₁ addition group. | |

BrCG inhibited apoptosis- suppressing activity by PGE₁. As a result, the effect that PGE₁ suppressed apoptosis inducted by GSNO in a rat renal tubule epithelial cell line (NRK52E cells) was demonstrated to generate through PGT.

### [Experiment 9]

1) Drugs having suppressing activity on apoptosis inducted in kidney cells by an NO donor were investigated. PGK₁ and bicyclo PGE₂ were used as test drugs, but other manners were in accordance with Experiment 8. The final concentration of the test drugs was 1 µM. The n was 5. The results are indicated in Table 13.

**[Table 13]**

| Drug | Incidence of apoptosis (%) |
|---|---|
| Vehicle (GSNO treatment only) | 13±1 |
| PGK₁ | 8±1** |
| Bicyclo PGE₂ | 6±1** |

| | |
|---|---|
| Values are expressed as means±SEM. ** Indicates a critical value of less than 1% as a significant difference with respect to the vehicle during detection in Dunnet's test. | |

PGK and bicyclo PGE₂ suppressed apoptosis induced by GSNO in kidney cells.
2) It was investigated the effects of a PGT inhibitor on the apoptosis- suppressing activity of PGK₁ and bicyclo PGE₂ in kidney cells. With a final concentration of BrCG, which is a PGT inhibitor, of 60 µM, manners other than simultaneous addition of GSNO and test drugs were in accordance with the aforementioned methods in 1). The n was 4. The results are indicated in Tables 14 and 15.

**[Table 14]**

| Agent | Incidence of apoptosis (%) |
|---|---|
| No addition (vehicle; GSNO treatment only) | 22±2 |
| PGK₁ | 8±1 |
| PGK₁ + BrCG | 12±1** |
| Values are expressed as means±SEM. | |

| | |
|---|---|
| **Indicates a critical value of less than 1% in the t-test as a significant difference in. relation to the PGE₁ addition group. | |

**[Table 15]**

| Agent | Incidence of apoptosis (%) |
|---|---|
| No addition (vehicle; GSNO treatment only) | 22±2 |
| Bicyclo PGE₂ | 9±2 |
| Bicyclo PGE₂ + BrCG | 16±3* |
| Values are expressed as means±SEM. | |

| | |
|---|---|
| *Indicates a critical value of less than 1% in the t-test as a significant difference in relation to the bicyclo PGE₂ addition group. | |

BrCG partially inhibited apoptosis-suppressing effect of PGK₁, bicyclo PGE₂.

### Industrial applicability

According to the present invention, cell apoptosis is suppressed by using substances having the property of uptake into cells through PGT, so said substances may be useful as cytoprotectants. They may be useful in particular as suppressor of neuron apoptosis, neuroprotectants, suppressor of kidney cell apoptosis, and cytoprotectants in kidney cell. Applications for prevention and/or treatment of neurological disorders, disorders accompanied by neurodegeneration, Alzheimer's disease, Parkinson's disease, Huntington's chorea, amyotrophic lateral sclerosis, and spinal canal stenosis are anticipated.

Moreover, according to the present invention, apoptosis is inducedby using substances having the property of PGT-inhibiting activityandmayallowapplicationinpreventionand/ortreatment of disorders involving cell proliferation of tumors and the like.

Furthermore, according to the present invention, it was determined that there is a relationship between cell apoptosis and signals arising through PGT, and SAPK/JNK is involved in this. Namely, substances in the present invention are taken into cells through PGT, and these substances suppress cell apoptosis by inducing of inhibitory intercellular signals or indicating inhibitory effect to SAPK/JNK activation. The suppression of cell apoptosis is anticipated to show cytoprotective activity.

Furthermore, according to the method of screening in the present invention, simple screening of substances showing cell apoptosis-inducing activity and substances showing cytoprotective activity with uptake into cells through PGT may be possible.

## Claims

1. A method of screening apoptosis-regulating substances **characterized by** screening of candidate substances through activity on prostaglandin transporter (PGT).

2. A method of screening apoptosis-regulating substances as claimed in claim 1, wherein the activity on PGT is measured by the amount of uptake into cells through PGT.

3. A method of screening apoptosis-regulating substances as claimed in claim 1, wherein the activity on PGT is measured by the rate of uptake into cells through PGT.

4. A method of screening apoptosis-regulating substances as claimed in claim 1, wherein the activity on PGT is measured by inhibition of PGT expression or PGT activity.

5. A method of screening apoptosis-regulating substances as claimed in any claim of claims 1-4, which comprises screening substances having substantially no hypotensive effect.

6. New apoptosis-regulating substances as screened by the method claimed in any claim of claims 1-5.

7. Cytoprotectants being composed of, as an active ingredient, apoptosis-regulating substances having activity to suppress apoptosis and having activity for uptake into cells through PGT.

8. Cytoprotectants as claimed in claim 7, wherein the activity for uptake into cells through PGT is displayed by an amount of uptake of at least about 70fmol/mg protein/10 mins.

9. Cytoprotectants as claimed in claim 7, wherein the activity for uptake into cells through PGT, have affinity for PGT as displayed by a permeation velocity (Km) of no more than about 100nm.

10. Cytoprotectants as claimed in any claim of claims 7-9, wherein the cells are kidney cells, neuron, or brain cells.

11. Cytoprotectants as claimed in any claim of claims 7-10, which have substantially no hypotensive activity.

12. Apoptosis-inducing agents being composed of, as an active ingredient, apoptosis-regulating substances having activity to induce apoptosis and having an inhibiting effect of PGT expression or PGT activity.

13. Apoptosis-inducing agents as claimed in claim 12, wherein the apoptosis-regulating substance is anti-PGT antibodies or PGT antisense.

14. Cytoprotectants as claimed in any claim of claims 7-11, wherein the apoptosis-regulating substance is selected from PGK₁, PGK₂, or bicyclo PGE₂.

15. A method of culturing PGT-expressing cells, which comprises using culture medium with an added cytoprotectant being composed of, as an active ingredient, apoptosis-regulating substances having activity to suppress apoptosis.

16. A method of culturing as claimed in claim 15, wherein the cytoprotectant has activity for uptake into cells through PGT.

17. A method of culturing as claimed in claim 15 or 16, wherein the cytoprotectant is PGE₁.

18. A method of regulating apoptosis which comprises administering an effective dose of an apoptosis-inducing agent or a cytoprotectant as claimed in any claim of claims 7-12, 13, or 14.

19. Use of an apoptosis-inducing agent or cytoprotectant as claimed in any claim of claims 7-12, 13, or 14 for manufacturing of medicaments for apoptosis regulation.
